# EUROPEAN PATENT SPECIFICATION

(11) **EP 1 850 807 B1**
(45) Date of publication and mention of the grant of the patent: **18.10.2017**
(21) Application number: 06735569.3
(22) Date of filing: 21.02.2006
(51) Int. Cl.: A61F 2/44

(54) **POLYAXIAL ORTHOPEDIC FASTENING APPARATUS**
MEHRACHSIGE ORTHOPÄDISCHE BEFESTIGUNGSVORRICHTUNG
DISPOSITIF DE FIXATION ORTHOPEDIQUE POLYAXIAL

(30) Priority: 22.02.2005 US 63941; 19.12.2005 US 312323
(43) Date of publication of application: 07.11.2007
(73) Proprietor: Facet Solutions, Inc., Logan, UT 84321 (US)
(72) Inventor: HOY, Robert W., Columbus, Ohio 43221 (US); FALLIN, T. Wade, Hyde Park, Utah 84318 (US); JUSTIN, Daniel F., Logan, Utah 84321 (US); MEIBOS, David W., Draper, Utah 84020 (US)
(74) Representative: HGF Limited
(86) International application number: PCT/US2006/005975
(87) International publication number: WO 2006/091539

(56) References cited:
- WO-A1-00/15125
- US-A- 4 805 602
- US-A- 6 063 090
- US-A1- 2004 006 391
- US-B2- 6 669 729

## Description

### 1. The Field of the Invention

The present invention relates generally to systems and methods for attaching implants to bone, and more specifically, to a polyaxial orthopedic fastening apparatus particularly useful in the field of facet joint replacement.

### 2. The Relevant Technology

Orthopedic medicine provides a wide array of implants that can be attached to bone to alleviate various pathologies. One unique challenge in orthopedics is to provide implants and fastening devices that are adaptable to a variety of bone morphologies. Each patient will have a different bone structure; accordingly, it may be necessary to allow for adjustable positioning of an implant with respect to the bone so that the implant will be positioned to perform its function.

For this reason, a number of fixation systems have been invented that enable variation of the angle between the implant and the fastener. Although such fixation systems generally permit adaptation to the bone morphology of a patient to provide secure anchoring of the implant to bone, they are generally somewhat limited in the types of adjustment they permit with respect to the bone. Accordingly, such fixation systems may not be usable with a number of implants that require more comprehensive adjustability. Furthermore, many known implant fixation systems are complex due to the presence of several parts, or due to the need to perform several steps to utilize them to attach an implant to bone. Yet further, some known implant fixation systems are expensive, and require the use of unusual tooling. A need exists in the art for implant fixation systems and methods that alleviate the foregoing shortcomings.

WO 00/15125 discloses a variable angle spinal fixation system. The system includes a longitudinal member positionable along a spinal column, a fastener having a threaded end for engaging a vertebra, and a connector member for connecting the fastener and the longitudinal member. The connector member has a channel extending through side surfaces of the connector member for receiving the longitudinal member, an opening laterally displaced from the channel and extending through top and bottom surfaces of the connector member for receiving the fastener, and a fastener clamping element for securing the fastener in the opening at a surgeon selected angle relative to the connector member and longitudinal member. This system allows angulation of the fastener to accommodate complex pathologies.

The invention provides an apparatus according to claim 1.

The dependent claims retout optional features of the apparatus.

### BRIEF DESCRIPTION OF THE DRAWINGS

Various embodiments of the present invention will now be discussed with reference to the appended drawings. It is appreciated that these drawings depict only typical embodiments of the invention and are therefore not to be considered limiting of its scope.
Figure 1 is an exploded, perspective view of a vertebra with an apparatus according to one embodiment of the invention, with the apparatus positioned for attachment to the vertebra.
Figure 2 is a caudal, section view of the vertebra and the apparatus of Figure 1, with the orthopedic fastener of the apparatus in the unlocked configuration to permit adjustment of the orientation of the implant against the bone.
Figure 3 is a caudal, section view of the vertebra and apparatus of Figure 1, with the orthopedic fastener in the locked configuration to restrict rotational motion of the implant against the bone.
Figure 4 is an exploded, perspective view of a vertebra with an apparatus according to one alternative embodiment of the invention, with the apparatus positioned for attachment to the vertebra.
Figure 5 is an exploded, perspective view of a vertebra with an apparatus according to an example, with the apparatus positioned to attach an implant to the vertebra.
Figure 6 is a perspective view of the vertebra with the apparatus of Figure 5 secured to the vertebra in the locked configuration to lock both rotation and translation of the implant.
Figure 7 is a cephalad, section view of the vertebra with the apparatus of Figure 5 secured to the vertebra in the locked configuration as in Figure 6.

### DETAILED DESCRIPTION

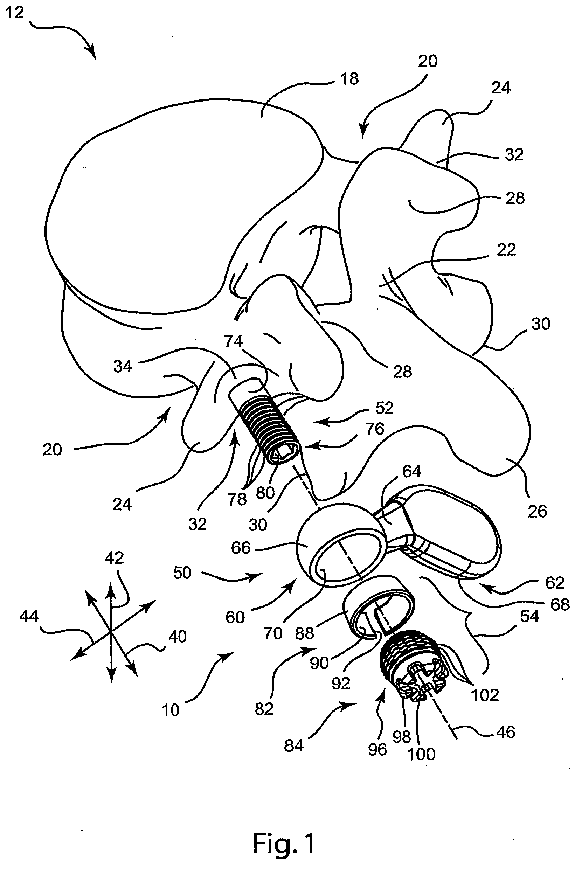

The present invention advances the state of the art by providing systems and methods that can be used to anchor orthopedic implants to bone in a manner that provides a high degree of implant adjustability, simplicity, and ease of use. The present invention can be used in any orthopedic procedure, but may have particular utility in the field of facet joint replacement to alleviate back pain resulting from traumatic, inflammatory, metabolic, synovial, neoplastic and degenerative spinal disorders. The configuration and operation of selected embodiments of the invention will be shown and described in greater detail with reference to Figures 1 through 7, as follows.

In this application, the terms "fastener," "engagement member," "compression member," and "interpositional member" are used broadly. A "fastener" generally relates to one or more members that can be used to "lock" two other objects together by restricting relative rotation and/or translation about or along at least one axis. More precisely, a "rotational fastener" is a fastener that restricts relative rotation of the two objects. A "translational" fastener is a fastener that restricts relative translation of the two objects. An "engagement member" is a member that is movable into and/or out of contact with another member to accomplish a function such as locking the members together. A "compression member" generally is a member that receives a compressive force. An "interpositional member" generally is a member, at least part of which is designed to be positioned between at least two other members of a system. Deformation of one part "to lock" another part generally relates to deformation of the first part in such a manner that the first part physically impedes relative motion between the two parts, rather than simply supplying frictional force to provide locking. However, the present invention encompasses embodiments that provide locking solely via one or more frictional forces.

"Polyaxial" rotation is rotation that can occur about at least two axes that are not parallel to each other. "Triaxial rotation" is rotation about three perpendicular axes. Triaxial rotation is equivalent to rotation about a point, because free rotation about any axis of a 3D coordinate system is the same as rotation that is not limited to any axis in the system.

Referring to Figure 1, a perspective view illustrates an apparatus 10 according to one embodiment of the invention, in use with a vertebra 12, such as an L5 lumbar vertebra of a human spine. As shown, the vertebra 12 has a body 18, which is generally disc-shaped. The vertebra 12 also has two pedicles 20 extending from the body 18, and a posterior arch, or lamina 22, that extends between the posterior ends of the pedicles 20 to couple the pedicles 20 together. The vertebra 12 also has a pair of transverse processes 24 that extend laterally from the pedicles 20, and a spinous process 26 that extends posteriorly from the lamina 22.

The vertebra 12 also has a pair of superior facets 28, which are positioned toward the top of the vertebra 12 and face generally medially. Additionally, the vertebra 12 has inferior facets 30, which are positioned toward the bottom of the vertebra 12 and face generally laterally. Each of the pedicles 20 of the vertebra 12 has a saddle point 32, which is positioned generally at the center of the juncture of each superior facet 28 with the adjacent transverse process 24.

The superior facets 28 of the vertebra 12 articulate (*i.e.,* slide and/or press) against the inferior facets (not shown) of an adjacent superior vertebra (not shown) to limit relative motion between the vertebra 12 and the superior vertebra. Thus, the combination of each superior facet 28 with the adjacent inferior facet defines a facet joint (not shown). Accordingly, two facet joints span the distance between each adjacent pair of vertebrae. The inferior facets 30 of the vertebra 30 are part of other facet joints that control motion between the vertebra 12 and an adjacent inferior vertebra (not shown) and/or the sacrum (also not shown).

Each of the facet joints may be covered by a capsule (not shown) containing a fluid (not shown) that reduces wear of the facets 28, 30 and facilitates articulation. Additionally, layers of cartilage (not shown) may cover the facets 28, 30 to further reduce wear and facilitate articulation. These anatomical structures, as well as the various muscles, ligaments, and nerves of the spine, will not be depicted in the Figures to enhance the clarity of the disclosure. Such structures may be removed or displaced according to known methods to provide the necessary access to the vertebra 12.

As shown, a semispherical resection 34 has been formed on one of the saddle points 32 of the vertebra 12. The semispherical resection 34 is shaped to receive an implant to replace the articular surface of one or both of the adjacent superior and inferior facets 28, 30. The semispherical resection 34 permits relative rotation between the implant and the vertebra 12 about three perpendicular axes prior to fixation of the implant to the vertebra 12. The axes may be defined as shown by reference numerals 40, 42, and 44 in Figure 1.

More precisely, the axes may include a first axis 40, a second axis 42, and a third axis 44. The first axis 40 is generally collinear with the axis of the corresponding pedicle 20. The second axis 42 is generally vertical (*i.e*., parallel to the axis of the body 18) and perpendicular to the first axis 40. The third axis 44 is generally horizontal (*i.e.,* parallel to the end plates of the body 18) and perpendicular to the first and second axes 40, 42.

The apparatus 10 includes an implant 50, a fixation member 52, and an orthopedic fastener 54, or fastener 54. The implant 50 is designed to seat against the semispherical resection 34 and to replace the articular surface of the inferior facet 30 immediately inferior to it. The fixation member 52 may take the form of a pedicle screw designed to be implanted in the corresponding pedicle 20 to anchor the implant 50 in place. The orthopedic fastener 54 is designed to be coupled to the fixation member 52 to hold the implant 50 against the vertebra 12.

In the embodiment of Figure 1, the implant 50 has a fixation portion 60, an articulation portion 62, and a stem 64. The fixation portion 60 is shaped to be attached to the semispherical resection 34, and the articulation portion 62 provides a surface that articulates with an adjacent vertebral facet to carry out the function of the inferior facet 30. The articulation portion 62 is coupled to the fixation portion 60 by the stem 64.

As shown, the fixation portion 60 has a bone apposition surface 66, which may be generally semispherical to correspond to the shape of the semispherical resection 34. The fixation portion 60 also has an aperture (not visible in Figure 1) that passes through the bone apposition surface 66 to receive the fixation member 52. The aperture is somewhat larger than the exterior surface of the fixation member 52 so that the bone apposition surface 66 is able to slide against the semispherical resection 34 with the fixation member 52 in place, implanted in the pedicle 20.

The articulation portion 62 similarly has an articulation surface 68 designed to articulate with a superior facet of a vertebra immediately inferior to the vertebra 12. The articulation surface 68 may have a convex shape, which may further be semispherical, semicylindrical, or the like. The articulation surface 68 may be designed to articulate with a natural superior facet or a prosthetic superior facet.

In addition to the bone apposition surface, the fixation portion 60 also has an interpositional interface 70 shaped to interact with the fastener 54 in a manner that will be described subsequently. The interpositional interface 70 has a generally conical surface that converges to the aperture of the fixation portion 60.

In the embodiment of Figure 1, the fixation member 52 has a distal end 74 implanted into one of the pedicles 20 of the vertebra 12, and a proximal end 76 that protrudes from the corresponding saddle point 32. The distal end 74 has threads (not visible in Figure 1) that facilitate implantation of the distal end 74 in the pedicle 20 and keep the implanted distal end 74 in place. The proximal end 76 has a plurality of threads 78 that are exposed to receive the fastener 54. Additionally, the proximal end 76 has a torquing interface that may be used to apply torque to the fixation member 52 to implant the distal end 74 in the pedicle 20. The torquing interface may take the form of a hexagonal recess into which a hexagonal driver end can be inserted.

As shown in Figure 1, the fastener 54 includes an interpositional member 82 and a compression member 84. The interpositional member 82 may take the form of a split ring, as illustrated. The compression member 84 is designed to be advanced along the proximal end 76 of the fixation member 52 to press the interpositional member 82 into the interpositional interface 70 of the implant 50.

More specifically, the interpositional member 82 has an implant interface 88, a compression interface 90, and a gap 92. As embodied in Figure 1, the implant interface 88 includes a generally conical exterior surface designed to mate with the interpositional interface 70 in such a manner that the interpositional member 82 is compressed inward relatively uniformly as it is urged into the interpositional interface 70. The compression interface 90 includes a generally semispherical interior surface designed to receive a corresponding surface of the compression member 84. The gap 92 enables the interpositional member 82 to obtain a relatively high degree of deflection to provide two very distinct configurations: an unlocked configuration, in which the interpositional member 82 is relatively undeflected, and a locked configuration in which the interpositional member 82 is compressed to narrow or remove the gap 92.

The interpositional member 82 may be formed of a material with a relatively low stiffness, such as plastic or rubber. A low stiffness provides relatively high deflection in the interpositional member 82 without requiring excessive force. Accordingly, moving the fastener 54 between the unlocked and locked configurations is relatively easily accomplished.

The compression member 84 has an interpositional interface 96, a torquing interface 98, and a bore 100. The interpositional interface 96 is shaped to mate with the compression interface 90 of the interpositional member 82. More specifically, the interpositional interface 96 may include an exterior, semispherical surface positionable within the compression interface 90. The torquing interface 98 may comprise a castle nut interface, with a plurality of radial projections that can be engaged by the end of a tool (not shown) with projections that mesh with the torquing interface 98. The torquing interface 98 is able to receive torque from such a tool to enable the compression member 84 to be advanced along the proximal end 76 of the fixation member 52.

The bore 100 passes through the compression member 84 and is sized to receive the proximal end 76. The bore 100 has threads (not shown) that engage the threads 78 of the proximal end 76 such that the compression member 84 advances along the proximal end 76 in response to rotation of the compression member 84.

The compression interface 90 and the interpositional interface 96 are sized such that they mate together with clearance when the interpositional member 82 is relatively undeflected. Thus, the interpositional member 82 and the compression member 84 are able to rotate with respect to each other about all three axes 40, 42, 44. Accordingly, when the interpositional member 82 is relatively undeflected or less deflected, the fastener 54 is in an unlocked configuration. When the interpositional member 82 is compressed, the compression interface 90 tightly engages the interpositional interface so that relative rotation about all three axes 40, 42, 44 is restricted. Thus, when the interpositional member 82 is relatively compressed, the fastener 54 is in a locked configuration.

The interpositional interface 96 may have features, such as a plurality of ridges 102, that are designed to engage the interpositional member 82 in the locked configuration to enhance locking. The ridges 102 extend around the circumference of the interpositional interface 96, about the first axis 40. Each of the, ridges 102 has a faceted shape, as shown in Figure 1, so that each ridge 102 has multiple relatively sharp projections that extend outward form the generally semispherical shape of the interpositional interface 96.

The compression member 84 may be formed of a material harder than that of the interpositional member 82. For example, the compression member 84 may be formed of a biocompatible metal. Accordingly, the projections of the ridges 102 may embed themselves into the compression interface 90 when the fastener 54 is moved to the locked configuration to enhance locking by resisting relative rotation between the interpositional member 82 and the compression member 84.

The interpositional interface 96 and the compression interface 90 may be relatively sized such that, in the unlocked configuration, there is little enough clearance that the interpositional member 82 and the compression member 84 will generally remain assembled. Thus, the interpositional member 82 and the compression member 84 may be pre-assembled (*i.e*., factory assembled or the like), so that the fastener 54 is ready for use at the commencement of the surgical procedure without further assembly.

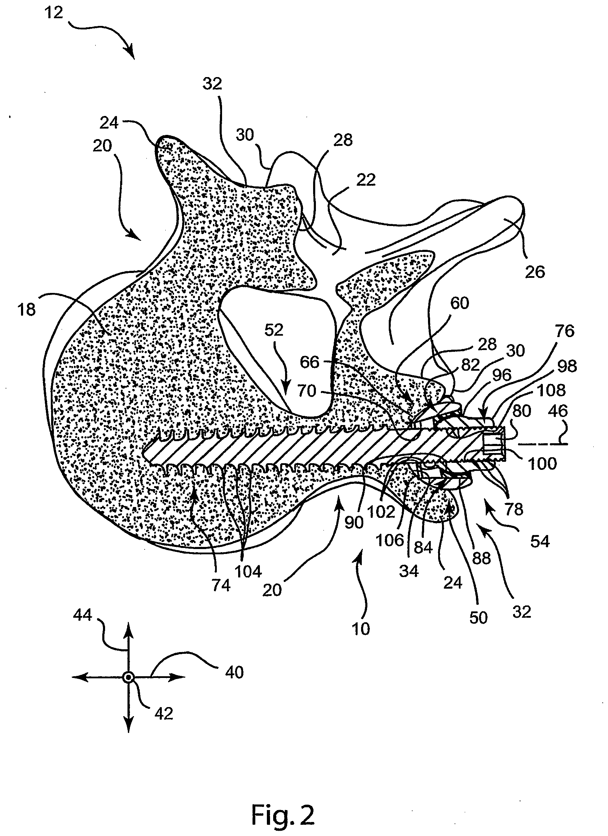

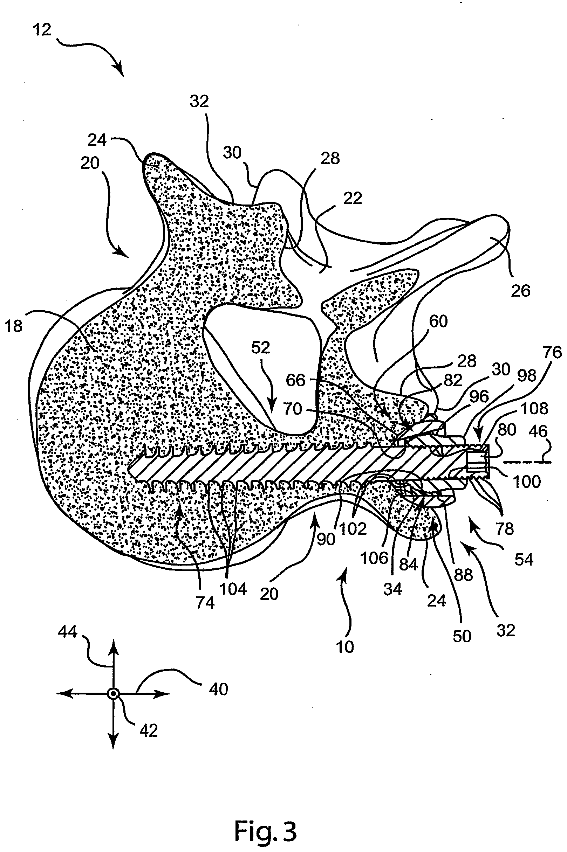

The fastener 54 may be used in concert with the fixation member 52 to retain the implant 50 in any of a plurality of orientations with respect to the vertebra 12. The manner in which this is carried out will be shown and described in greater detail with reference to Figures 2 and 3, as follows.

Referring to Figure 2, a cephalad, section view illustrates the apparatus 10 and the vertebra 12, with the apparatus 10 fully assembled and with the fastener 54 in the unlocked configuration. As shown, the distal end 74 of the fixation member 52 has threads 104 that are embedded in the body 18 and in the corresponding pedicle 20 of the vertebra 12. The fixation portion 60 of the implant 50 has an aperture 106 to which the generally conical surface of the interpositional interface 70 of the implant 50 converges. The bore 100 of the compression member 84 has threads 108 that engage the threads 78 of the proximal end 76 of the fixation member 52. These elements were briefly described in the discussion of Figure 1, but were not visible in Figure 1.

In order to install the implant 50, the posterior elements of the vertebra 12 may first be exposed through the use of procedures known in the art. Measurements may be made to select the implant 50 and determine the appropriate orientation for the implant 50, and any other implants to be installed in the same operation. The semispherical resection 34 may be formed via a reaming operation or the like, and other portions of the vertebra 12, such as the inferior facet 30 to be replaced, may be resected as needed. The fixation member 52 may be implanted in the corresponding pedicle 20 along the desired angle.

The implant 50 may then be inserted and positioned such that the bone apposition surface 66 abuts the semispherical resection 34. Prior to adjustment of the orientation of the implant 50, the fastener 54 may be installed so that it can be used to easily fix the implant 50 in place once it has reached the desired orientation. As mentioned previously, the interpositional member 82 and the compression member 84 may be pre-assembled, and therefore, they may not need to be assembled prior to implantation.

The compression member 84, with the interpositional member 82 already coupled to it, may be positioned so that the proximal end 76 of the fixation member 52 passes into the bore 100 of the compression member 84. The compression member 84 may then be rotated to cause the threads 108 of the bore 100 to engage the threads 78 of the proximal end 76. The interpositional member 82 is inserted into the interpositional interface 70 of the implant 50 such that the generally conical implant interface 88 of the interpositional member 82 engages the generally conical surface of the interpositional interface 70. The interpositional member 82 is thereby drawn into coaxiality with the fixation portion 60 of the implant 50.

Further rotation of the compression member 84 will urge the interpositional member 82 further along the interpositional interface 70, toward the pedicle 20. The engagement of the generally conical surfaces of the interpositional interface 70 and the implant interface 88 causes the interpositional member 82 to contract in response to such motion, thereby bringing the fastener 54 to the locked configuration. Until the implant 50 has been adjusted to the desired orientation, the compression member 84 may be rotated just far enough to keep it in place to facilitate subsequent tightening, but not far enough to move the fastener 54 to the locked configuration.

Thus, the apparatus 10 reaches the configuration shown in Figure 2. The fastener 54 is coupled to the proximal end 76 of the fixation member 52, but has not been tightened. Accordingly, although the orientation of the interpositional member is substantially fixed with respect to the implant 50, the compression member 84 is rotatable about any of the axes 40, 42, 44 with respect to the interpositional member 82. Accordingly, the implant 50 is still relatively freely rotatable about the axes 40, 42, 44 with respect to the vertebra 12.

The implant 50 may then be adjusted by rotating the implant 50 with respect to the vertebra 12 such that the bone apposition surface 66 rotates about any or all of the axes 40, 42,44 within the semispherical resection 34, until the articulation surface 68 is at the desired position and angle. Then, it is desirable to fix the orientation of the implant 50 with respect to the bone, as will be shown and described in connection with Figure 3.

Referring to Figure 3, a cephalad, section view illustrates the apparatus 10 and the vertebra 12, with the fastener 54 in the locked configuration. After rotational adjustment of the implant 50 with respect to the vertebra 12, the compression member 84 is further rotated, for example, by engaging the torquing interface 98 with a suitable tool, as described previously, and applying torque. The compression member 84 is advanced along the proximal end 76, toward the pedicle 20 so that the interpositional member 82 is compressed by engagement with the interpositional interface 70 of the implant 50.

As the interpositional member 82 is compressed, the gap 92 shrinks and the compression interface 90 of the interpositional member 82 contracts to grip the interpositional interface 96. The ridges 102, or just the projections thereof, embed themselves in the compression interface 90 to restrict and/or entirely prevent further rotation of the compression member 84 with respect to the interpositional member 82. The interpositional interface 70 of the implant 50 and the implant interface 88 of the interpositional member 82 engage each other in such a manner that relative rotation between the implant 50 and the interpositional member 82 is substantially prevented about the second and third axes 42, 44. Relative rotation about the first axis 40 is also restricted as the interpositional member 82 is compressed, and therefore, provides frictional force as it presses outward against the interpositional interface 70.

Accordingly, when the compression member 84 is advanced along the proximal end 76 to provide the locked configuration, relative rotation between the vertebra 12 and the implant 50 are substantially prevented. This is the configuration illustrated in Figure 3. The implant 50 is locked in its preferred orientation with respect to the vertebra 12. The implant 50 can be fixed in any of a wide variety of different orientations with respect to the vertebra 12 through the use of the fixation member 52 and the fastener 54.

The embodiment shown in Figures 1, 2, and 3 is only one of many embodiments of the present invention. In alternative embodiments, differently shaped implants, interpositional members, compression members, and fixation members may be used. For example, generally conical surfaces need not be used to interface between an implant and an interpositional member. Such an interfacing surface may include cylindrical, semispherical, conical, parabolic, or other shapes, or combinations thereof. According to one alternative embodiment, an interfacing surface man include a cylindrical component adjoining a semicylindrical or parabolic component that flares the interface to provide a diameter larger than that of the cylindrical component. Such an interfacing surface may also have a polygonal cross section, or may be keyed or otherwise shaped to limit an implant to a discrete number of relative orientations with respect to a vertebra.

Similarly, generally semispherical surfaces need not be used to interface between the interpositional member and the compression member. Such interfacing surfaces may include cylindrical, semispherical, conical, parabolic, or other shapes, or combinations thereof. By contrast with the embodiment of Figures 1, 2, and 3, alternative embodiments of the invention need not necessarily provide rotation of an implant with respect to a vertebra about three perpendicular axes.

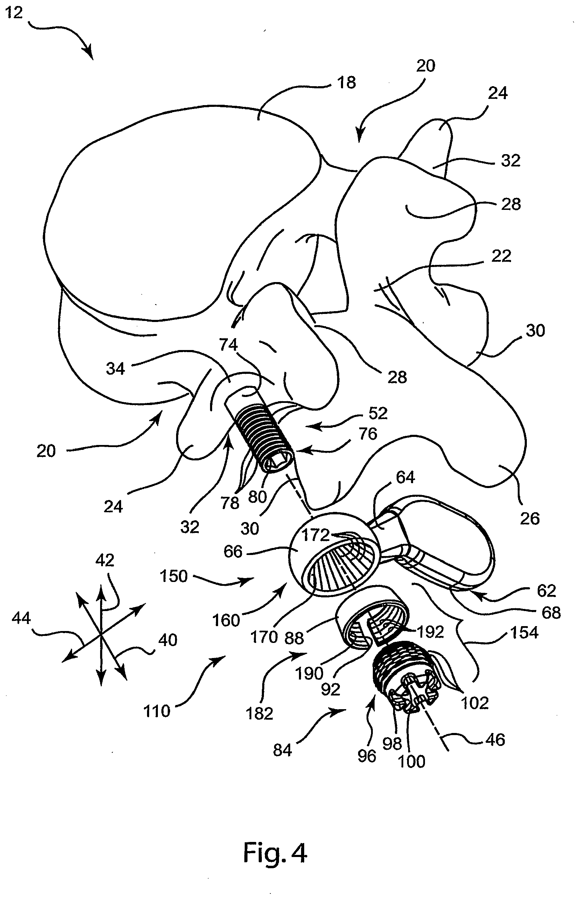

In some alternative embodiments, additional features may be added to enhance locking provided by the locked configuration. Such features may enable an implant to receive greater loads without moving from its preferred orientation with respect to the vertebra. One example of such an alternative embodiment will be shown and described in connection with Figure 4, as follows.

Referring to Figure 4, a perspective view illustrates an apparatus 110 according to one alternative embodiment of the invention, used in conjunction with a vertebra 12, as described previously. As shown, the apparatus 110 includes an implant 150, a fixation member 52, and an orthopedic fastener 154, or fastener 154. The fixation member 52 may be identical to that of the previous embodiment. The implant 150 and the fastener 154 are similar to their counterparts of the previous embodiment, but include additional features to enhance locking, as will be described below.

As shown, the implant 150 has a fixation portion 160 designed to be attached to the semispherical resection 34. The fixation portion 160 has an interpositional interface 170, which has a generally conical surface like that of the interpositional interface 70 of the previous embodiment. However, the interpositional interface 170 does not provide a smooth conical surface. Rather, the interpositional interface 170 has a plurality of ridges 172 distributed along a generally radially symmetrical pattern about the generally conical surface.

The ridges 172 protrude inward to resist relative rotation between the implant 150 and an interpositional member 182 of the fastener 154. Like the interpositional member 82 of the previous embodiment, the interpositional member 182 has an implant interface 88 with a generally conical shape. The implant interface mates with the interpositional interface 170 of the implant 150. By contrast with the previous embodiment, the ridges 172 of the interpositional interface 170 are able to penetrate the generally conical surface of the implant interface 88 to restrict, or even substantially prevent, relative rotation between the implant 150 and the interpositional member 182 about the first axis 40.

Penetration of the implant interface 88 by the ridges 172 may occur because the implant 150 may be formed of a material that is harder than that of the interpositional member 182. For example, the interpositional member 182 may be formed of a polymer, elastomer, or the like, while the implant 150 may be formed of a metal, a ceramic, or a harder polymer or elastomer. Thus, the ridges 172 may serve to enhance the locking provided by the locked configuration of the fastener 154.

In addition to the interpositional member 182, the fastener 154 has a compression member 84, which is identical to that of the previous embodiment. However, the interpositional member 182 differs from the interpositional member 82 of the previous embodiment in that the interpositional member 182 has a compression interface 190 with features designed to enhance locking with the interpositional interface 96 of the compression member 84. More precisely, the compression interface 190 includes a generally semispherical surface like that of the previous embodiment. However, the compression interface 190 has a plurality of ridges 192 that extend along generally circular pathways about the circumference of the of the compression interface 190.

In the locked configuration, the ridges 192 enhance locking by helping prevent rotation between the compression interface 190 and the interpositional interface 96 of the compression member 84. More precisely, the ridges 192 may deform against the interpositional interface 96 of the compression member 84, thereby providing regions in which the frictional force between the compression interface 190 and the interpositional interface 96 is relatively large. The ridges 192 may also directly block motion of the ridges 102 of the interpositional interface 96, thereby particularly restricting relative rotation between the interpositional member 182 and the compression member 84 about the second and third axes 42, 44.

Thus, the ridges 172 and the ridges 192 that have been added in the apparatus 110 of Figure 4 may serve to enhance locking of the orientation of the implant 150 with respect to the vertebra 12, in the locked configuration of the fastener 154. Those of skill in the art will recognize that alternative features may be added to enhance locking. In selected alternatives, the ridges 172 of the interpositional interface 170 of the implant 150 may follow non-converging or curvilinear pathways designed to avoid interfering with compression of the interpositional member 182. Furthermore, the differently-shaped surfaces described previously for interfacing between the implant 50 and the interpositional member 82 or between the interpositional member 82 and the compression member 84 may be used in conjunction with features like the ridges 172 and the ridges 192 of the embodiment of Figure 4 to provide enhanced locking for such alternative interfacing surface shapes.

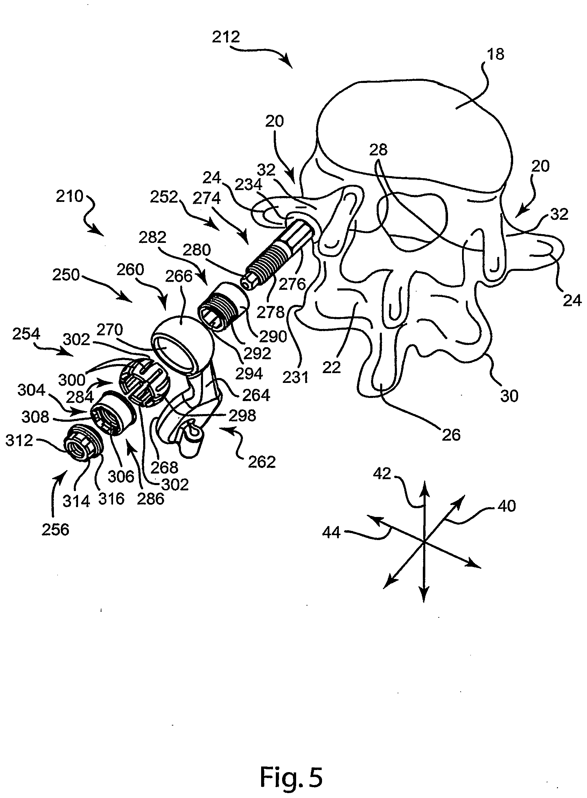

Referring to Figure 5, a perspective view illustrates an apparatus 210, in use with a vertebra 212, such as an L4 lumbar vertebra of a human spine. Like the vertebra 12, the vertebra 212 has a body 18, which is generally disc-shaped. The vertebra 212 also has two pedicles 20 extending from the body 18, and a posterior arch, or lamina 22, which extends between the posterior ends of the pedicles 20 to couple the pedicles 20 together. The vertebra 212 also has a pair of transverse processes 24 that extend laterally from the pedicles 20, and a spinous process 26 that extends posteriorly from the lamina 22.

The vertebra 212 also has a pair of superior facets 28, which are positioned toward the top of the vertebra 12 and face generally medially. Additionally, the vertebra 212 has an inferior facet 30, which is positioned toward the bottom of the vertebra 12 and faces generally laterally. A resected inferior facet 231 also faces generally laterally. The articular surface of the resected inferior facet 231 may optionally have been resected away to prepare the resected inferior facet 231 for arthroplasty. Each of the pedicles 20 of the vertebra 212 has a saddle point 32, which is positioned generally at the center of the juncture of each superior facet 28 with the adjacent transverse process 24.

The superior facets 28 of the vertebra 212 articulate *(i.e.,* slide and/or press) against the inferior facets (not shown) of an adjacent superior vertebra (not shown), such as an L3 lumbar vertebra, to limit relative motion between the vertebra 12 and the superior vertebra. Thus, the combination of each superior facet 28 with the adjacent inferior facet defines a facet joint (not shown). Accordingly, two facet joints span the distance between each adjacent pair of vertebrae. The inferior facets 30 of the vertebra 212 are part of other facet joints that control motion between the vertebra 12 and an adjacent inferior vertebra (not shown), such as an L5 lumbar vertebra or the sacrum.

Each of the facet joints may be covered by a capsule (not shown) containing a fluid (not shown) that reduces wear of the facets 28, 30 and facilitates articulation. Additionally, layers of cartilage (not shown) may cover the facets 28, 30 to further reduce wear and facilitate articulation. These anatomical structures, as well as the various muscles, ligaments, and nerves of the spine, will not be depicted in the Figures to enhance the clarity of the disclosure. Such structures may be removed or displaced according to known methods to provide the necessary access to the vertebra 212.

As shown, a semispherical resection 34 has been formed on one of the saddle points 32 of the vertebra 212. The semispherical resection 34 is shaped to receive an implant to replace the articular surface of one or both of the adjacent superior and inferior facets 28, 30. The semispherical resection 34 permits relative rotation between the implant and the vertebra 212 about three perpendicular axes prior to fixation of the implant to the vertebra 212. The axes may be defined as shown by the reference numerals 40, 42, and 44, which correspond to those of Figure 1.

The apparatus 210 includes an implant 250, a fixation member 252, a rotational fastener 254, and a translational fastener 256. The implant 250 is designed to seat against the semispherical resection 234 and to replace the removed articular surface of the resected inferior facet 231 immediately inferior to it. The fixation member 252 may take the form of a pedicle screw designed to be implanted in the corresponding pedicle 20 to anchor the implant 250 in place. The orthopedic fastener 254 is designed to be coupled to the fixation member 252 to hold the implant 250 against the vertebra 212.

In the example of Figure 5, the implant 250 has a fixation portion 260, an articulation portion 262, and a stem 264. The fixation portion 260 is shaped to be attached to the semispherical resection 234, and the articulation portion 262 provides a surface that articulates with an adjacent vertebral facet to carry out the function of the inferior facet 30. The articulation portion 262 is coupled to the fixation portion 260 by the stem 264.

As shown, the fixation portion 260 has a bone apposition surface 266, which may be generally semispherical to correspond to the shape of the semispherical resection 234. The fixation portion 260 also has an aperture (not visible in Figure 5) that passes through the bone apposition surface 266 to receive the fixation member 252. The aperture is somewhat larger than the exterior surface of the fixation member 252 so that the bone apposition surface 266 is able to slide against the semispherical resection 234 with the fixation member 252 in place, implanted in the pedicle 20.

The articulation portion 262 similarly has an articulation surface 268 designed to articulate with a superior facet of a vertebra (or sacrum) immediately inferior to the vertebra 212. The articulation surface 268 may have a convex shape, which may further be semispherical, semicylindrical, or the like. The articulation surface 268 may be designed to articulate with a natural superior facet and/or a prosthetic superior facet.

In addition to the bone apposition surface 266, the fixation portion 260 also has an engagement surface 270 shaped to receive the rotational fastener 254 such that the rotational fastener 254 is able to restrict relative rotation between the implant 250 and the fixation member 252. The engagement surface 270 has a generally semispherical concave shape through which the aperture (not shown) of the fixation portion 260 passes.

In the example of Figure 5, the fixation member 252 has a distal end (not visible in Figure 5) 274 implanted into the corresponding pedicle 20 of the vertebra 212, and a proximal end 274 that protrudes from the corresponding saddle point 32. The distal end has threads that facilitate implantation of the distal end 274 in the pedicle 20 and keep the implanted distal end in place. The fixation member 252 also has a sliding interface 276 positioned between the distal end and the proximal end 276. The sliding interface 276 may have a polygonal or other non-circular cross section shaped to receive the rotational fastener 254 in such a manner that no significant relative rotation can occur between the sliding interface 276 and the rotational fastener 254.

The proximal end 274 has a plurality of threads 278 that are exposed to receive the fastener 254. Additionally, the proximal end 274 has a torquing interface 280 that may be used to apply torque to the fixation member 252 to implant the distal end in the pedicle 20. The torquing interface 280 may take the form of a hexagonal recess or projection that mates with a corresponding hexagonal feature on a driver (not shown).

As shown, the rotational fastener 254 includes an interpositional member 282, an engagement member 284, and a rotational locking member 286. The interpositional member 282 may have a generally tubular shape with a tapered portion 290, a plurality of threads 292 adjacent to the tapered portion 290, and an interface 294. As shown, the tapered portion 290 becomes narrower toward the threads 292. The interface 294 is designed to provide a slidable, yet non-rotating connection between the interpositional member 282 and the sliding interface 276 of the fixation member 252. Accordingly, the interface 294 may take the form of a bore with a polygonal cross section that receives the corresponding polygonal cross section of the sliding interface 276 with enough clearance to permit relatively free sliding motion. Alternatively, a tighter fit may be used to restrict sliding, but permit relative translation between the interpositional member 282 and the fixation member 252 under the application of force.

As also illustrated in Figure 5, the engagement member 284 is generally spherical in shape, with a hollow interior. The hollow interior has a taper that generally matches the taper of the tapered portion 290 of the interpositional member 282. The engagement member 284 has an implant engagement surface 298 with a semispherical shape, and a plurality of grooves 300 arranged in a parallel, substantially radially symmetrical fashion about the implant engagement surface 298. The grooves 298 permit expansion and contraction of the implant engagement surface 298. The hollow interior is accessible via ports 302 positioned at either end of the implant engagement surface 298.

The rotational locking member 286 has a bore 304 in which a plurality of threads 306 are formed. The threads 306 are designed to mate with the threads 292 of the interpositional member 282. The bore 304 also has a torquing interface 308 formed therein to facilitate rotation of the rotational locking member 286 into engagement with the interpositional member 282. The torquing interface 308 may take the form of a portion of the bore 304 having a generally polygonal cross sectional shape, such as a hegaxonal cross sectional shape. Thus, a corresponding hexagonal protrusion of a driver (not shown) may be inserted into the torquing interface 308 to rotate the rotational locking member 86 into engagement with the interpositional member 282.

The translational fastener 256, which may also be termed a translational locking member, has a threaded bore 312, a torquing interface 314, and a flange 316. The threads of the threaded bore 312 are sized to rotate into engagement with the threads 278 of the proximal end 274 of the fixation member 252. The torquing interface 314 may fake the form of a protrusion having a generally hexagonal shape capable of being received within a recess of a driver (not shown) having a corresponding hexagonal shape.

The flange 316 protrudes generally radially from the exterior of the translational fastener 256, adjacent to the torquing interface 314. The flange 316 may be sized to abut the adjoining annular surface of the rotational locking member 286 to enable the translational fastener 256 to exert relatively uniform, linear force against the rotational locking member 286 upon tightening of the translational fastener 256. If desired, a portion of the translational fastener 256 may nest within the bore 304 of the rotational locking member 286 to reduce the profile of the assembled apparatus 210.

The apparatus 210 may be secured to the vertebra 212 according to a variety of methods. According to one method, the fixation member 252 is first implanted in the corresponding pedicle 20. This may be carried by, for example, forming an incision in the overlying tissues, retracting the tissues from the operating area, implanting a guide wire in the pedicle 20 under fluoroscopy, and then rotating the fixation member 252 into engagement with the pedicle 20 through the use of a driver (not shown) coupled to the torquing interface 280.

Through the use of the rotational fastener 254 and the translational fastener 256, the orientation of the implant 250 and the position of the implant 250 along the fixation member 252 (*i.e*., along the first axis 40) may be independently locked. The rotational fastener 254 and the implant 250 may first be assembled together by assembling the interpositional member 282, the engagement member 284, the rotational locking member 286, and the implant 250.

The engagement member 284 may first be inserted into the hollow interior of the fixation portion 260 of the implant 250. Since the engagement member 284 has not yet been significantly expanded, there is clearance between the implant engagement surface 298 of the engagement member 284 and the engagement surface 270 of the fixation portion 260 of the implant 250. This clearance permits rotation of the engagement member 284 within the fixation portion 260. The engagement surface 270 of the fixation portion 260 may have a semispherical shape that extends far enough to effectively capture the engagement member 284. Accordingly, the engagement member 284 may need to be compacted and/or pressed into the hollow interior of the fixation portion 260.

The interpositional member 282 is then inserted through the aperture (not shown) of the fixation portion 260 and into the hollow interior of the engagement member 284 such that the tapered portion 290 extends through the port 302 that will be oriented toward the fixation member 252, and the threads 292 extend through the other port 302 (i.e., the port 302 that will be oriented toward the translational fastener 256 and the rotational locking member 286, as shown in the exploded view of Figure 5). Then, the rotational locking member 286 is positioned adjacent to the corresponding port 302 such that the threads 292 enter the bore 304.

The rotational locking member 286 is rotated with respect to the interpositional member 282 such that the threads 306 of the bore 304 engage the threads 292 of the interpositional member 282. Continued rotation of the rotational locking member 286 with respect to the interpositional member 282 will cause the engagement member 284 to expand as the opposite port 302 slides toward the larger end of the tapered portion 290. However, at this stage, the rotational fastener 254 remains in the unlocked configuration because the rotational locking member 286 is only rotated sufficiently to engage the threads 292 to keep the rotational locking member 286, the interpositional member 282, and the engagement member 284 together.

The assembled implant 250 and rotational fastener 254 may then be advanced toward the proximal end 274 of the implanted fixation member 252 such that the torquing interface 280, and then at least some of the threads 278, pass through the interface 294, or bore, of the interpositional member 282. The interface 294 then slides around the sliding interface 276 of the proximal end 274 of the fixation member 252. As mentioned before, some clearance may exist between the sliding interface 276 of the proximal end 274 and the interface 294 of the interpositional member 282. However, the matching polygonal shapes of the sliding interface 276 and the interface 294 prevent relative rotation between the fixation member 252 and the rotational fastener 254.

Since the rotational fastener 254 is still in the unlocked configuration, the implant 250 may be rotated with respect to the fixation member 252 and the vertebra 212. The implant 250 is pivoted generally about the center of the radius of the engagement surface 270 until the articulation surface 268 is properly positioned and oriented to articulate with the corresponding natural or prosthetic superior articulation surface. In the embodiment of Figure 5, rotation of the implant 250 is not only polyaxial, but also triaxial. Thus, the implant 250 may be rotated about any axis passing through the center of the radius of the engagement surface 270.

Once the implant 250 has been rotated into the proper orientation with respect to the vertebra 212, it may be locked in that orientation by moving the rotational fastener 254 to the locked configuration. The rotational locking member 286 is further rotated with respect to the interpositional member 282, for example, by engaging the torquing interface 308 of the bore 304 with a corresponding feature of a driver (not shown). This rotation urges the opposite port 302 to advance along the tapered portion 290 of the interpositional member 282, toward the larger end of the tapered portion 290. The outward pressure on the port 302 causes the engagement member 284 to expand, thereby increasing the overall radius of the implant engagement surface 298. The implant engagement surface 298 engages the engagement surface 270 of the fixation portion 260 of the implant and exerts outward pressure on the engagement surface 270. As a result, the implant 250 becomes locked to the engagement member 284.

Thus, the rotational fastener 254 has reached the locked configuration, and the implant 250 is no longer rotatable with respect to the vertebra 212. However, the implant 250, together with the rotational fastener 254 that is now rigidly locked to it, may still move along the fixation member 252. The translational fastener 256 may then be applied to restrict such translational motion. More precisely, the translational fastener 256 is moved toward the proximal end 274 of the fixation member 252 such that the threads 278 of the proximal end 274 enter the threaded bore 312 of the translational fastener 256. The translational fastener 256 is rotated to advance the threaded bore 312 along the threads 278 until the flange 316 presses snugly against the adjoining annular surface of the rotational locking member 286. This effectively presses the bone apposition surface 266 of the fixation portion 260 of the implant 250 against the semispherical resection 234 of the corresponding saddle point 32.

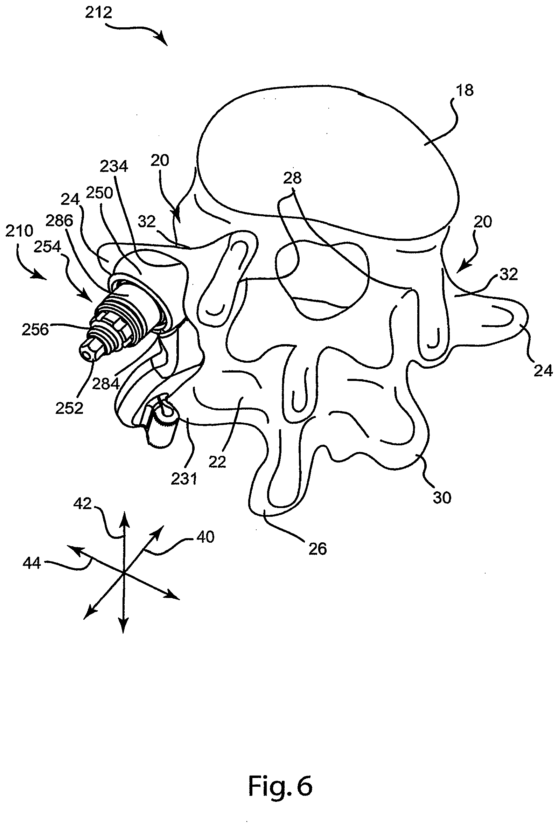

Referring to Figure 6, a perspective view illustrates the apparatus 210 in fully assembled form on the vertebra 212. The position and orientation of the implant 250 are fixed with respect to the vertebra 212. Advantageously, since the orientation and position of the implant 250 are independently locked, any subsidence of the bone around the saddle point 32 will not enable the implant 250 to rotate from its desired orientation with respect to the vertebra 212. If such subsidence occurs, the position of the implant 250 may be stabilized with relatively simple revision surgery, *i.e.,* by further tightening the translational fastener 256 or by inserting bone graft, an implant, or some other form of support into the space between the bone apposition surface 266 and the semispherical resection 234.

Those of skill in the art will recognize that an apparatus similar to the apparatus 210 may be applied to the opposite side of the vertebra 212 for bilateral operation. The fixation member 252, rotational fastener 254, and translational fastener 256 may be used to attach left or right, superior and/or inferior, implants to the vertebra 212. In alternative examples (not shown), similar components to the components 252, 254, and 256 may even be used to secure nested fixation portions of superior and inferior implants to a single saddle point 32. In yet other alternative examples, such similar components may be used to secure other types of implants to the vertebra 212 besides facet joint implants, including but not limited to artificial discs, posterior rod fixation systems, dynamic stabilization systems, and the like (not shown).

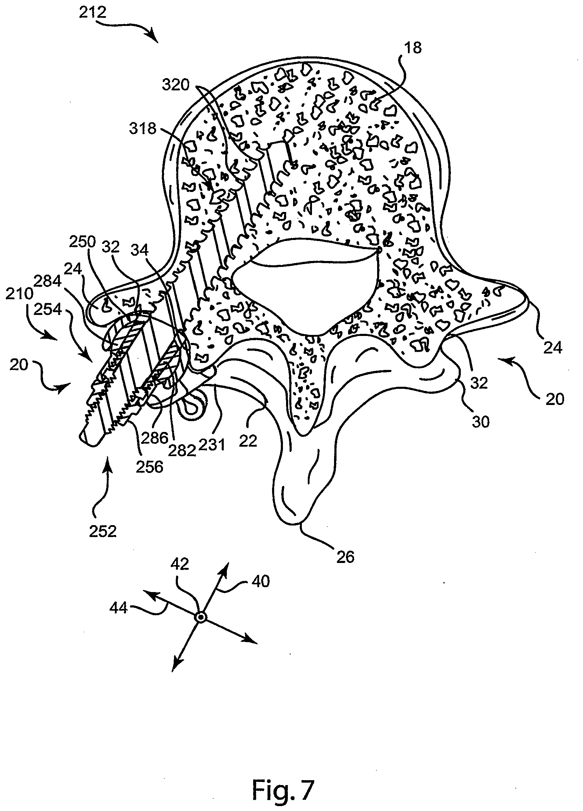

Referring to Figure 7, a cephalad, section view illustrates the apparatus 210 in fully assembled form on the vertebra 212. As mentioned previously, the fixation member 252 has a distal end 318 with threads 320 that engage the interior of the corresponding pedicle 20. Another potential advantage to independent rotational and translational locking of the implant 250 is that the purchase of the threads 320 within the pedicle 20 is not significantly challenged by any of the steps used to lock the orientation of the implant 250 with respect to the vertebra 212. Only the axial force exerted by locking of the translational fastener 256 is transmitted to the interface between the threads 320 and the surrounding bone. This decreases the probability that the bone between the threads 320 will fail under shear and permit the distal end 318 to pull free of the bone.

The present invention has particular relevance to orthopedic medicine, and more particularly to facet joint replacement. However, the principles and structures of the present invention may also be extended to a wide variety of other fields.

The described embodiments are to be considered in all respects only as illustrative and not restrictive. The scope of the invention is indicated by the appended claims rather than by the foregoing description.

## Claims

1. An apparatus comprising:
an implant (50) comprising a bone apposition surface (66) shaped to slide against a corresponding surface of a bone to permit rotation of the implant with respect to the bone about a first axis (40); and
an orthopedic fastener (54) configured to secure the implant to the bone, the orthopedic fastener comprising:
a compression member (84); and
an interpositional member (82) configured to cooperate with the compression member (84) to provide an unlocked configuration in which the interpositional member is rotatable along the first axis (40) and two other axes (42, 44) with respect to the compression member, and a locked configuration in which relative rotation between the interpositional member (82) and the compression member (84) is restricted;
wherein the orthopedic fastener (54) is configured to move from the unlocked configuration to the locked configuration in response to urging of the compression member toward the bone along a direction nonparallel to the first axis (40) to fix an orientation of the implant (50) with respect to the bone about the first axis (40).

2. The apparatus of claim 1, further comprising a fixation member (52) implantable in the bone, the fixation member (52) having a receiving interface configured to cooperate with the compression member (84) to urge the compression member toward the bone.

3. The apparatus of claim 2, wherein the fixation member (52) comprises a distal end (74) shaped to be implanted in the bone, and a proximal end (76) having threads (78), wherein the compression member (84) comprises threads shaped to cooperate with the threads (78) of the proximal end (76) to cause the compression member to advance toward the distal end in response to rotation of the compression member with respect to the fixation member.

4. The apparatus of claim 1, wherein the implant comprises an articular surface (68) shaped to replace a natural articular surface of a facet of the vertebra.

5. The apparatus of claim 1, wherein the bone apposition surface (66) comprises a generally semispherical shape.

6. The apparatus of claim 1, wherein at least one of the compression member (84) and the interpositional member (82) is configured to deform in response to motion of the compression member (84) toward the bone to lock against the other of the compression member (84) and the "interpositional member (82).

7. The apparatus of claim 6, wherein at least one of the compression member (84) and the interpositional member (82) comprises a plurality of features that protrude toward the other of the compression member (84) and the interpositional member (82) to restrict relative rotation between the compression member and the interpositional member.

8. The apparatus of claim 6, wherein the interpositional member (82) comprises a split ring configured to contract in response to motion of the compression member toward the bone.

9. The apparatus of claim 1, wherein the interpositional member (82) comprises an implant interface having a generally conical shape, wherein the implant comprises an interpositional interface (70) having a generally conical shape that mates with the implant interface to restrict rotation of the implant with respect to the bone about the first axis (40).

10. The apparatus of claim 9, wherein at least one of the implant interface and the interpositional interface comprises a plurality of features that protrude toward the other of the implant interface and the interpositional interface (70) to restrict relative rotation between the implant and the interpositional member.

11. The apparatus of claim 1, wherein the bone apposition surface (66) is further shaped to slide against the corresponding surface of the bone to permit rotation of the implant with respect to the bone about a second axis (42) perpendicular to the first axis, wherein motion of the orthopedic fastener from the unlocked configuration to the locked configuration further fixes an orientation of the implant with respect to the bone about the second axis (42).

12. The apparatus of claim 2, wherein:
the interpositional member (182) comprises a locking surface and an implant interface (88), wherein the locking surface engages the compression member to provide the unlocked configuration in which the interpositional member (182) is rotatable with respect to the compression member (84) about an axis, and the locked configuration in which the relative rotation is restricted about the axis, and the implant interface is shaped to engage the implant to press the implant toward the bone in response to urging of the compression member toward the bone; and
the fixation member (52) is implantable in the bone along a direction nonparallel to the axis in the unlocked configuration.

13. The apparatus of claim 12, wherein the implant comprises an articular surface (70) shaped to replace a natural articular surface of a facet of a vertebra.

14. The apparatus of claim 12, wherein the fixation member (52) comprises a distal end (74) shaped to be implanted in the bone, and a proximal end (76) having threads (78), wherein the compression member (84) comprises threads shaped to cooperate with the threads (78) of the proximal end (76) to cause the compression member (84) to advance toward the distal end in response to rotation of the compression member (84) with respect to the fixation member (52).

15. The apparatus of claim 12, wherein at least one of the compression member (84) and the interpositional member (82) is configured to deform in response to motion of the compression member (84) toward the bone to lock against the other of the compression member (84) and the interpositional member (82).

16. The apparatus of claim 15, wherein at least one of the compression member (84) and the interpositional member (182) comprises a plurality of features that protrude toward the other of the compression member (84) and the interpositional member (182) to restrict relative rotation between the compression member (84) and the interpositional member (182).

17. The apparatus of claim 12, wherein the interpositional member comprises an implant interface having a generally conical shape, wherein the implant comprises an interpositional interface having a generally conical shape that mates with the implant interface to restrict rotation of the implant with respect to the bone about the axis.

## Patentansprüche

1. Vorrichtung, die Folgendes umfasst:
ein Implantat (50), das eine Knochenappositionsfläche (66) umfasst, die dafür geformt ist, gegen eine entsprechende Fläche eines Knochens zu gleiten, um eine Drehung des Implantats im Verhältnis zum Knochen um eine erste Achse (40) zu ermöglichen; und
eine orthopädische Befestigungsvorrichtung (54), die dazu konfiguriert ist, das Implantat am Knochen zu befestigen, wobei die orthopädische Befestigungsvorrichtung Folgendes umfasst:
ein Kompressionselement (84); und
ein Zwischenelement (82), das dazu konfiguriert ist, mit dem Kompressionselement (84) zusammenzuwirken, um eine nicht-sperrende Konfiguration bereitzustellen, in der das Zwischenelement entlang der ersten Achse (40) und zwei weiterer Achsen (42, 44) im Verhältnis zum Kompressionselement gedreht werden kann, sowie eine sperrende Konfiguration, in der die relative Drehung zwischen dem Zwischenelement (82) und dem Kompressionselement (84) eingeschränkt ist;
wobei die orthopädische Befestigungsvorrichtung (54) dazu konfiguriert ist, aus der nicht-sperrenden in die sperrende Konfiguration zu wechseln, in Reaktion auf das Drücken des Kompressionselements in Richtung des Knochens entlang einer Richtung, die nicht parallel zur ersten Achse (40) verläuft, um die Ausrichtung des Implantats (50) im Verhältnis zum Knochen um die erste Achse (40) zu korrigieren.

2. Vorrichtung nach Anspruch 1, die weiterhin ein in den Knochen implantierbares Befestigungselement (52) umfasst, wobei das Befestigungselement (52) eine Empfangsschnittstelle aufweist, die dazu konfiguriert ist, mit dem Kompressionselement (84) zusammenzuwirken, um das Kompressionselement in Richtung des Knochens zu drücken.

3. Vorrichtung nach Anspruch 2, wobei das Befestigungselement (52) ein distales Ende (74), das für die Implantierung in den Knochen geformt ist, und ein proximales Ende (76), das Gewinde (78) aufweist, umfasst, wobei das Kompressionselement (84) Gewinde umfasst, die für das Zusammenwirken mit den Gewinden (78) des proximalen Endes (76) geformt sind, um das Kompressionselement in Reaktion auf die Drehung des Kompressionselements im Verhältnis zum Befestigungselement in Richtung des distalen Endes zu verschieben.

4. Vorrichtung nach Anspruch 1, wobei das Implantat eine Gelenkfläche (68) umfasst, die dafür geformt ist, eine natürliche Gelenkfläche einer Facette des Wirbels zu ersetzen.

5. Vorrichtung nach Anspruch 1, wobei die Knochenappositionsfläche (66) eine im Wesentlichen halbkugelförmige Form umfasst.

6. Vorrichtung nach Anspruch 1, wobei zumindest eines von Kompressionselement (84) und Zwischenelement (82) dazu konfiguriert ist, sich in Reaktion auf eine Bewegung des Kompressionselements (84) in Richtung des Knochens zu verformen, um gegen das andere von Kompressionselement (84) und Zwischenelement (82) zu sperren.

7. Vorrichtung nach Anspruch 6, wobei zumindest eines von Kompressionselement (84) und Zwischenelement (82) eine Vielzahl von Elementen umfasst, die in Richtung des anderen von Kompressionselement (84) und Zwischenelement (82) hervorstehen, um die relative Drehung zwischen dem Kompressionselement und dem Zwischenelement einzuschränken.

8. Vorrichtung nach Anspruch 6, wobei das Zwischenelement (82) einen Spaltring umfasst, der dazu konfiguriert ist, sich in Reaktion auf die Bewegung des Kompressionselements in Richtung des Knochens zusammenzuziehen.

9. Vorrichtung nach Anspruch 1, wobei das Zwischenelement (82) eine Implantatsschnittstelle mit einer im Wesentlichen kegelförmigen Form umfasst, wobei das Implantat eine Zwischenschnittstelle (70) mit einer im Wesentlichen kegelförmigen Form umfasst, die sich an die Implantatsschnittstelle anpasst, um die Drehung des Implantats im Verhältnis zum Knochen um die erste Achse (40) einzuschränken.

10. Vorrichtung nach Anspruch 9, wobei zumindest eine von Implantatsschnittstelle und Zwischenschnittstelle eine Vielzahl von Elementen umfasst, die in Richtung der anderen von Implantatsschnittstelle und Zwischenschnittstelle (70) hervorstehen, um die relative Drehung zwischen dem Implantat und dem Zwischenelement einzuschränken.

11. Vorrichtung nach Anspruch 1, wobei die Knochenappositionsfläche (66) weiterhin dafür geformt ist, gegen die entsprechende Fläche des Knochens zu gleiten, um die Drehung des Implantats im Verhältnis zum Knochen um eine zweite Achse (42), die senkrecht zur ersten Achse verläuft, zu ermöglichen, wobei die Bewegung der orthopädischen Befestigungsvorrichtung aus der nicht-sperrenden Konfiguration in die sperrende Konfiguration weiterhin die Ausrichtung des Implantats im Verhältnis zum Knochen um die zweite Achse (42) korrigiert.

12. Vorrichtung nach Anspruch 2, wobei:
das Zwischenelement (182) eine Sperrfläche und eine Implantatsschnittstelle (88) umfasst, wobei die Sperrfläche mit dem Kompressionselement in Eingriff kommt, um die nicht-sperrende Konfiguration, in der das Zwischenelement (182) im Verhältnis zum Kompressionselement (84) um eine Achse gedreht werden kann, und die sperrende Konfiguration, in der die relative Drehung um die Achse eingeschränkt ist, bereitzustellen, und die Implantatsschnittstelle dafür geformt ist, mit dem Implantat in Eingriff zu kommen, um das Implantat in Reaktion auf das Drücken des Kompressionselements in Richtung des Knochens in Richtung des Knochens zu drücken; und
das Befestigungselement (52) entlang einer Richtung in den Knochen implantiert werden kann, die nicht parallel zur Achse in der nicht-sperrenden Konfiguration verläuft.

13. Vorrichtung nach Anspruch 12, wobei das Implantat eine Gelenkfläche (70) umfasst, die dafür geformt ist, eine natürliche Gelenkfläche einer Facette eines Wirbels zu ersetzen.

14. Vorrichtung nach Anspruch 12, wobei das Befestigungselement (52) ein distales Ende (74), das für die Implantierung in den Knochen geformt ist, und ein proximales Ende (76), das Gewinde (78) aufweist, umfasst, wobei das Kompressionselement (84) Gewinde umfasst, die für das Zusammenwirken mit den Gewinden (78) des proximalen Endes (76) geformt sind, um das Kompressionselement (84) in Reaktion auf die Drehung des Kompressionselements (84) im Verhältnis zum Befestigungselement in Richtung des distalen Endes zu verschieben (52).

15. Vorrichtung nach Anspruch 12, wobei zumindest eines von Kompressionselement (84) und Zwischenelement (82) dazu konfiguriert ist, sich in Reaktion auf eine Bewegung des Kompressionselements (84) in Richtung des Knochens zu verformen, um gegen das andere von Kompressionselement (84) und Zwischenelement (82) zu sperren.

16. Vorrichtung nach Anspruch 15, wobei zumindest eines von Kompressionselement (84) und Zwischenelement (182) eine Vielzahl von Elementen umfasst, die in Richtung des anderen von Kompressionselement (84) und Zwischenelement (182) hervorstehen, um die relative Drehung zwischen dem Kompressionselement (84) und dem Zwischenelement (182) einzuschränken.

17. Vorrichtung nach Anspruch 12, wobei das Zwischenelement eine Implantatsschnittstelle mit einer im Wesentlichen kegelförmigen Form umfasst, wobei das Implantat eine Zwischenschnittstelle mit einer im Wesentlichen kegelförmigen Form umfasst, die sich an die Implantatsschnittstelle anpasst, um die Drehung des Implantats im Verhältnis zum Knochen um die Achse einzuschränken.

## Revendications

1. Appareil comprenant :
un implant (50) comprenant une surface d'apposition osseuse (66) formée pour coulisser contre une surface correspondante d'un os afin de permettre la rotation de l'implant par rapport à l'os autour d'un premier axe (40) ; et
un élément de fixation orthopédique (54) conçu pour fixer solidement l'implant à l'os, l'élément de fixation orthopédique comprenant :
un élément de compression (84) ; et
un élément d'interposition (82) conçu pour coopérer avec l'élément de compression (84) pour fournir une configuration déverrouillée dans laquelle l'élément d'interposition peut tourné le long du premier axe (40) et de deux autres axes (42, 44) par rapport à l'élément de compression et une configuration verrouillée dans laquelle la rotation relative entre l'élément d'interposition (82) et l'élément de compression (84) est limitée ;
ledit élément de fixation orthopédique (54) étant conçu pour passer de la configuration déverrouillée à la configuration verrouillée en réponse à la poussée de l'élément de compression vers l'os selon une direction non parallèle au premier axe (40) afin de fixer une orientation de l'implant (50) par rapport à l'os autour du premier axe (40).

2. Appareil selon la revendication 1, comprenant en outre un élément de fixation (52) implantable dans l'os, ledit élément de fixation (52) possédant une interface de réception conçue pour coopérer avec l'élément de compression (84) pour pousser l'élément de compression vers l'os.

3. Appareil selon la revendication 2, ledit élément de fixation (52) comprenant une extrémité distale (74) formée pour être implantée dans l'os, et une extrémité proximale (76) possédant des filetages (78), l'élément de compression (84) comprenant des taraudages formés pour coopérer avec les filetages (78) de l'extrémité proximale (76) pour amener l'élément de compression à avancer vers l'extrémité distale en réponse à une rotation de l'élément de compression par rapport à l'élément de fixation.

4. Appareil selon la revendication 1, ledit implant comprenant une surface articulaire (68) formée pour remplacer une surface articulaire naturelle d'une facette de la vertèbre.

5. Appareil selon la revendication 1, ladite surface d'apposition osseuse (66) comprenant une forme généralement hémisphérique.

6. Appareil selon la revendication 1, au moins l'un de l'élément de compression (84) et de l'élément d'interposition (82) étant conçu pour se déformer en réponse au mouvement de l'élément de compression (84) vers l'os pour se verrouiller contre l'autre de l'élément de compression (84) et de l'élément d'interposition (82).

7. Appareil selon la revendication 6, au moins l'un de l'élément de compression (84) et de l'élément d'interposition (82) comprenant une pluralité d'éléments qui font saillie vers l'autre de l'élément de compression (84) et de l'élément d'interposition (82) afin de limiter une rotation relative entre l'élément de compression et l'élément d'interposition.

8. Appareil selon la revendication 6, ledit élément d'interposition (82) comprenant un anneau fendu conçu pour se contracter en réponse au mouvement de l'élément de compression vers l'os.

9. Appareil selon la revendication 1, ledit élément d'interposition (82) comprenant une interface d'implant possédant une forme généralement conique, ledit implant comprenant une interface d'interposition (70) possédant une forme généralement conique qui s'accouple avec l'interface d'implant afin de limiter une rotation de l'implant par rapport à l'os autour du premier axe (40).

10. Appareil selon la revendication 9, au moins l'un de l'interface de l'implant et de l'interface d'interposition comprenant une pluralité d'éléments qui font saillie vers l'autre de l'interface d'implant et de l'interface d'interposition (70) afin de limiter une rotation relative entre l'implant et l'élément d'interposition.

11. Appareil selon la revendication 1, ladite surface d'apposition osseuse (66) étant en outre formée pour coulisser contre la surface correspondante de l'os pour permettre une rotation de l'implant par rapport à l'os autour d'un second axe (42) perpendiculaire au premier axe, ledit mouvement de l'élément de fixation orthopédique depuis la configuration déverrouillée jusqu'à la configuration verrouillée fixe en outre une orientation de l'implant par rapport à l'os autour du second axe (42).

12. Appareil selon la revendication 2 :
ledit élément d'interposition (182) comprenant une surface de verrouillage et une interface d'implant (88), ladite surface de verrouillage se mettant en prise avec l'élément de compression pour obtenir la configuration déverrouillée dans laquelle l'élément d'interposition (182) peut tourné par rapport à l'élément de compression (84) autour d'un axe et la configuration verrouillée dans laquelle la rotation relative est limitée autour de l'axe, et l'interface d'implant étant formée pour se mettre en prise avec l'implant afin de presser l'implant vers l'os en réponse à la poussée de l'élément de compression vers l'os ; et
ledit élément de fixation (52) étant implantable dans l'os selon une direction non parallèle à l'axe dans la configuration déverrouillée.

13. Appareil selon la revendication 12, ledit implant comprenant une surface articulaire (70) formée pour remplacer une surface articulaire naturelle d'une facette d'une vertèbre.

14. Appareil selon la revendication 12, ledit élément de fixation (52) comprenant une extrémité distale (74) formée pour être implantée dans l'os et une extrémité proximale (76) possédant des filetages (78), ledit élément de compression (84) comprenant des taraudages formés pour coopérer avec les filetages (78) de l'extrémité proximale (76) afin d'amener l'élément de compression (84) à avancer vers l'extrémité distale en réponse à une rotation de l'élément de compression (84) par rapport à l'élément de fixation (52).

15. Appareil selon la revendication 12, au moins l'un de l'élément de compression (84) et de l'élément d'interposition (82) étant conçu pour se déformer en réponse au mouvement de l'élément de compression (84) vers l'os pour se verrouiller contre l'autre de l'élément de compression (84) et de l'élément d'interposition (82).

16. Appareil selon la revendication 15, au moins l'un de l'élément de compression (84) et de l'élément d'interposition (182) comprenant une pluralité d'éléments qui font saillie vers l'autre de l'élément de compression (84) et de l'élément d'interposition (182) pour limiter la rotation relative entre l'élément de compression (84) et l'élément d'interposition (182).

17. Appareil selon la revendication 12, ledit élément d'interposition comprenant une interface d'implant possédant une forme généralement conique, ledit implant comprenant une interface d'interposition possédant une forme généralement conique qui s'accouple avec l'interface d'implant afin de limiter une rotation de l'implant par rapport à l'os autour de l'axe.
